# EUROPÄISCHE PATENTSCHRIFT

(11) **EP 0 412 282 B1**
(45) Veröffentlichungstag und Bekanntmachung des Hinweises auf die Patenterteilung: **31.05.1995**
(21) Anmeldenummer: 90112417.2
(22) Anmeldetag: 29.06.1990
(51) Int. Cl.: A61B 1/00, A61B 17/32, A61B 1/233

(54) **Endoskop für die nasale Chirurgie**
Endoscope for nasal surgery
Endoscope pour la chirurgie nasale

(30) Priorität: 19.07.1989 DE 3923851
(43) Veröffentlichungstag der Anmeldung: 13.02.1991
(73) Patentinhaber: Richard Wolf GmbH, 75438 Knittlingen (DE)
(72) Erfinder: Heckele, Helmut, D-7134 Knittlingen (DE)
(74) Vertreter: Wilcken, Thomas, Dipl.-Ing.

(56) Entgegenhaltungen:
- DE-A- 2 545 761
- DE-A- 3 233 564
- GB-A- 2 048 079
- US-A- 4 369 768
- Firmenprospekt der Fa. Richard Wolf GmbH, Knittlingen aus dem Jahr 1988 ("Saug-Spül-Handgriff zur Sinus-Chirurgie 8475.26")

## Beschreibung

Die Erfindung geht aus von einem Endoskop für die nasale Chirurgie (Sinuskop) nach dem Oberbegriff des Patentanspruches 1.

Ein derartiges Endoskop ist in der DE-C-38 03 212 beschrieben. Bei diesem bekannten Endoskop verläuft die Optik zentral so durch den Endoskopschaft, daß dadurch keine Möglichkeit besteht, einen zusätzlichen Instrumentenschaft für chirurgisch erforderliche Hilfsinstrumente optimal vorzusehen.

Die Aufgabe der Erfindung besteht bei Endoskopen der eingangs erwähnten Art darin, neben dem Zu- und Abführen einer Spülflüssigkeit zusätzlich ein Hilfsinstrument, z. B. eine Lasersonde, eine Zange oder dergleichen, durch das Endoskop für die nasale Chirurgie hindurchzuführen und distal unter visueller Kontrolle an die zu behandelnde Stelle in einer Körperhöhle heranführen zu können, und darin, das Hilfsinstrument sowohl für Links- als auch Rechtshänder leicht und bequem durch das Endoskop hindurchführen und handhaben zu können.

Diese Aufgabe wird durch die Kennzeichenmerkmale des Anspruches 1 gelöst.

Mit Hilfe des Arbeitseinsatzes ist es möglich, neben Schäften für die Spülung und Durchführung der Endoskopoptik einen gesonderten Schaft für die Durchführung eines Hilfsinstrumentes optimal vorzusehen. Dieser ist am proximalen Ende seiner Längsachse abgewinkelt und mit einem verdrehbaren, winkeligen Einführungsstutzen versehen. Der Einführungsstutzen kann somit nach links oder rechts zum Endoskophandgriff verschwenkt werden, wodurch die Handhabung und Einführung des Hilfsinstrumentes für Links- und Rechtshänder leicht und bequem durchgeführt werden kann.

Die Erfindung ist nachstehend anhand eines in der anliegenden Zeichnung dargestellten Ausführungsbeispieles näher erläutert. Es zeigen:

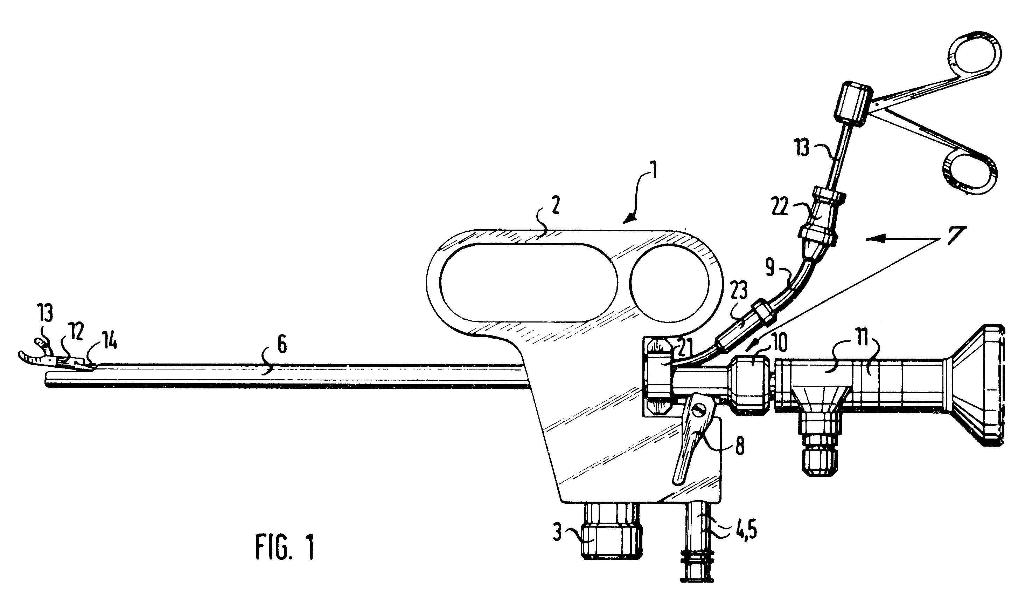

- Fig. 1: die Seitenansicht des Endoskopes für die nasale Chirurgie,

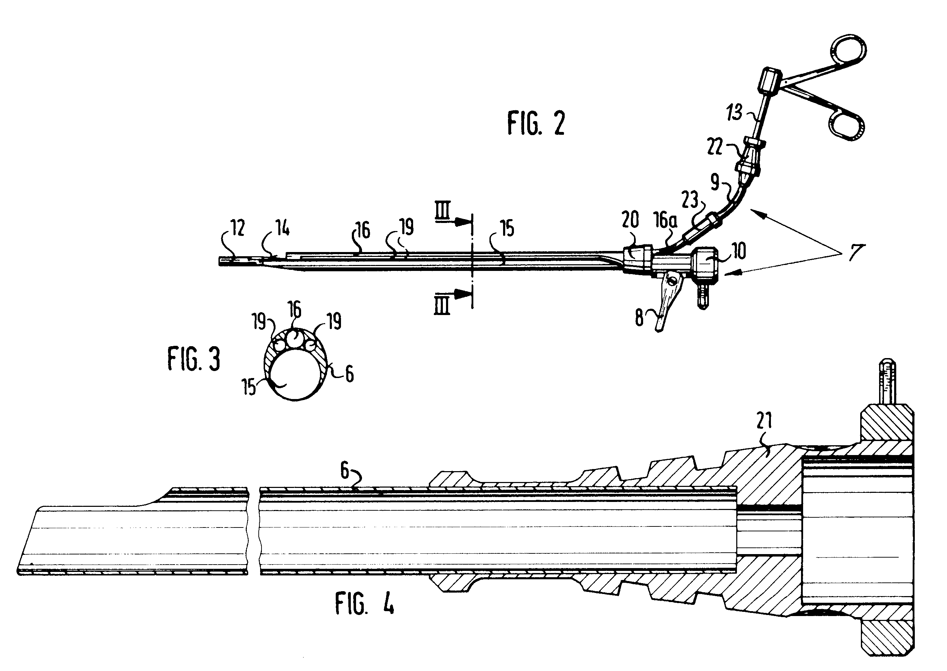

- Fig. 2: den Arbeitseinsatz für das Endoskop in Seitenansicht,
- Fig. 3: einen Querschnitt nach Linie III-III der Fig. 2, jedoch nach Einführen des Arbeitseinsatzes in
- Figur 4: den Außenschaft des Endoskopes.

Das erfindungsgemäße Endoskop bzw. Sinuskop 1 besteht nach der Zeichnung aus einer Handhabe 2 mit einem bekannten Umschaltventil 3 nach der DE-C 38 03 212 mit den Anschlußstutzen 4 und 5 und einem Außenschaft 6 mit einem Saug- und Spülschaft zum Zu- und Abführen von Spülflüssigkeiten oder dergleichen. Das Endoskop besteht weiterhin aus einem mit dem Außenschaft 6 proximal lösbar kuppelbaren Arbeitseinsatz 7 mit zwei beiseitigen Handhaben 8 und einem verschwenkbaren, winkeligen Instrumenteneinführungsstutzen 9 sowie aus einer am proximalseitigen Ende des Arbeitseinsatzes 7 mittels einer Kupplung 10 festlegbaren Optik 11. Zum Abwinkeln eines in die Körperhöhle einzuführenden Hilfsinstrumentes 13 weist der Arbeitseinsatz 7 an seinem distalen Ende einen schwenkbar beweglich angeordneten Albarran-Hebel 12 auf, mit dem das distale Ende des Hilfsinstrumentes 13 aus dem Außenschaft 6 heraus innerhalb eines begrenzten Schwenkwinkels abgelenkt werden kann.

Den wesentlichen Endoskopteil bildet der Arbeitseinsatz 7, der einen die Optik 11 aufnehmenden Schaft 15 aufweist, dessen distales, in Richtung auf einen ebenfalls in dem Einsatz 7 verlaufenden Instrumentenschaft 16 schräg nach oben verlaufendes, offenes Ende den Albarran-Hebel 12 oder ein anderes geeignetes Ablenkelement aufweist. Der Hebel 12 ist über eine oder mehrere Zug- und Druckstangen oder Drähte 14 mit den beiden, auf beiden Seiten des Arbeitseinsatzes 7 verdrehbaren Handhaben 8 und der mit diesen in Eingriff stehenden, axial verschiebbaren Stange (nicht dargestellt) direkt verbunden, um eine im Winkel zur Endoskoplängsachse erfolgende Schwenkbewegung ausführen zu können.

Die beiderseits des Instrumentenschaftes 16 verlaufenden Zug- und Druckstangen 14 verlaufen durch zwei mit dem Schaft 15 an dessen Oberseite unlösbar festgelegten Führungen 19. Um den hygienischen Anforderungen in erforderlichem Maße Rechnung zu tragen, sind der oval ausgebildete Außenschaft 6 des Endoskopes und der in diesen einführbare Arbeitseinsatz 7 über einen Kupplungskegel 20 und einen Spannring 21 lösbar miteinander verbindbar. Es besteht aber auch die Möglichkeit, diese beiden Teile unlösbar miteinander festzulegen, so daß diese eine untrennbare Einheit bilden.

Um die Handhabung des Hilfsinstrumentes 13 sowohl für einen Links- als auch für einen Rechtshänder leicht und bequem durchführen zu können, ist der Instrumentenschaft 16 proximal bei 16a seitlich abgewinkelt und mit dem winkeligen Einführungsstutzen 9 und einem Schlauchanschluß 22 versehen. Der Stutzen 9 ist in einem Lager 23 verdrehbar, so daß er sowohl zur linken als auch zur rechten Seite des Handgriffes 2 verschwenkbar ist, um eine Anpassung an einen Links- oder Rechtshänder zu erreichen und dadurch das Einführen und Bedienen des Hilfsinstrumentes 13 zu erleichtern. Außerdem kann zum gleichen Zweck der Albarran-Hebel 12 sowohl durch die eine Handhabe 8 auf der einen Endoskopseite oder die andere Handhabe 8 auf der anderen Endoskopseite betätigt werden.

## Patentansprüche

1. Endoskop für die nasale Chirurgie mit einem einen Saug- und Spülschaft aufweisenden Außenschaft (6) und mit einer mit diesem Schaft verbundenen Handhabe (2), in der ein Umschaltventil (3) für die Verbindung des Saug- und Spülschaftes mit einer Unterdruck- bzw. Spülmittelquelle und für die Unterbrechung dieser Verbindung vorgesehen ist, dadurch gekennzeichnet, daß mit dem Außenschaft (6) proximalwärts der Handhabe (2) ein durch den Außenschaft schiebbarer Arbeitseinsatz (7) gekuppelt ist, der einen Instrumentenschaft (16) für ein proximal durch einen abgewinkelten Stutzen (9) des Arbeitseinsatzes einführbares Hilfsinstrument (13), einen weiteren Schaft (15) für eine proximal an den Arbeitseinsatz (7) ankuppelbare Optik (11) und beidseitig verdrehbare Betätigungshandhaben (8) für Drähte oder Stangen (14) aufweist, welch letztere an einem zum Verschwenken des distalen Endes des Instrumentes (13) dienenden, distalen Hebel (12) angreifen, und daß das zur Längsachse des Endoskopes abgewinkelte proximale Ende (16a) des Instrumentenschaftes (16) für die Durchführung des Hilfsinstrumentes (13) mit einem Lager (23) für die Verdrehung des winkeligen Einführungsstutzens (9) für das Hilfsinstrument (13) versehen ist.

2. Endoskop nach Anspruch 1, dadurch gekennzeichnet, daß die Drähte oder Stangen (14) zum Verschwenken des distalen Hebels (12) in Führungen (19) vorgesehen sind, die zusammen mit dem Instrumentenschaft (16) für die Durchführung des Hilfsinstrumentes (13) mit dem weiteren Schaft (15) zur Bildung einer Einheit fest verbunden sind.

## Claims

1. An endoscope for nasal surgery with an outer shaft (6) having a suction- and flushing shaft, and with a handle (2) connected with this outer shaft, in which a changeover valve (3) is provided for the connection of the suction- and flushing shaft with a source of underpressure and flushing medium respectively and for the interruption of this connection, characterised in that a working insert (7) is coupled with the outer shaft (6) in proximal direction of the handle (2), which working insert (7) is able to be pushed through the outer shaft and has an instrument shaft (16) for an auxiliary instrument (13) able to be introduced proximally through an angled connecting piece (9) of the working insert, a further shaft (15) for an optical system (11) able to be coupled proximally to the working insert (7) and operating handles (8), rotatable on both sides, for wires or rods (14), which latter engage on a distal lever (12) serving to swivel the distal end of the instrument (13), and that the proximal end (16a) of the instrument shaft (16), angled to the longitudinal axis of the endoscope, is provided with a bearing (23) to pass the auxiliary instrument (13) through for the rotation of the angular introduction connecting piece (9) for the auxiliary instrument (13).

2. An endoscope according to Claim 1, characterised in that the wires or rods (14) are provided for swivelling the distal lever (12) in guideways (19), which together with the instrument shaft (16) for passing the auxiliary instrument (13) through are fixedly connected with the further shaft (15) to form one unit.

## Revendications

1. Endoscope pour la chirurgie nasale, avec une tige extérieure (6) présentant une tige d'aspiration et de balayage, et avec une poignée (2), reliée à cette tige et dans laquelle est prévue une soupape de commutation (3) destinée à la liaison de la tige d'aspiration et de balayage à une source de vide ou à une source de fluide de balayage et à interrompre cette liaison, caractérisé en ce qu'à la tige extérieure (6) est couplé, du côté proximal de la poignée (2), un insert de travail (7), pouvant être coulissé à travers la tige extérieure et présentant une tige pour instrument (16) destinée à un instrument auxiliaire (13), pouvant être introduit en position proximale à travers un embout (9) coudé de l'insert de travail, présentant une tige supplémentaire (15) pour une optique (11) pouvant être couplée en position proximale sur l'insert de travail (7) et des poignées d'actionnement (8) susceptibles de tourner, placées de part et d'autre, pour des fils métalliques ou des tiges (14), ces derniers agissant sur un levier distal (12) servant au pivotement de l'extrémité distale de l'instrument (13), et en ce que l'extrémité proximale (16a), coudée par rapport à l'axe longitudinal de l'endoscope, de la tige pour instrument (16) prévue pour le passage de l'instrument auxiliaire (13), est pourvue d'un palier (23) destiné à permettre la rotation de l'embout d'introduction (9) coudé destiné à l'instrument auxiliaire (13).

2. Endoscope selon la revendication 1, caractérisé en ce que les fils métalliques ou tiges (14) destinés à permettre le pivotement du levier distal (12) sont prévus dans des guidages (19) qui sont reliés rigidement à la tige supplémentaire (15), conjointement avec la tige pour instrument (16) destinée au passage de l'instrument auxiliaire (13), pour constituer un ensemble.
